Europäisches Patentamt

European Patent Office

Office européen des brevets

(11) Veröffentlichungsnummer: **0 263 193**
**A1**

## (12) EUROPÄISCHE PATENTANMELDUNG

(21) Anmeldenummer: **86113792.5**

(22) Anmeldetag: **04.10.86**

(51) Int. Cl.⁴ **A61N 5/06**

Die Bezeichnung der Erfindung wurde geändert (Richtlinien für die Prüfung im EPA, A-III, 7.3).

(43) Veröffentlichungstag der Anmeldung:
**13.04.88 Patentblatt 88/15**

(84) Benannte Vertragsstaaten:
**AT BE CH DE FR GB IT LI LU NL SE**

(71) Anmelder: **Helmut K. Pinsch GmbH & Co.**
**Hansastrasse 13**
**D-2000 Hamburg 13(DE)**

(72) Erfinder: **Hatje, Günter H.**
**Wellingsbüttler Weg 182**
**D-2000 Hamburg 65(DE)**

(74) Vertreter: **Richter, Joachim, Dipl.-Ing. et al**
**Patentanwälte Richter u.Werdermann Neuer**
**Wall 10**
**D-2000 Hamburg 36(DE)**

(54) **Verfahren und Vorrichtung zur Steigerung des Wohlbefindens eines Lebewesens.**

(57) Die Erfindung betrifft ein Verfahren zur Steigerung des Wohlbefindens eines Lebewesens, wobei eine Zielfläche auf der Haut des Lebewesens mit aus einer Laser-Lichtquelle stammenden Teilchen beaufschlagt wird. Die kurzgepulste Laser-Lichtquelle wird entfernt von der Zielfläche angeordnet und hat eine so hohe Austritts-Leistungsdichte, daß ihre Strahlung kurzzeitig eine hohe Leistung pro Flächeneinheit der Zielfläche auf die Zielfläche aufbringt.

EP 0 263 193 A1

## Verfahren zur Steigerung des Wohlbefindens eines Lebewesens.

Die Erfindung betrifft ein Verfahren zur Steigerung des Wohlbefindens eines Lebewesens, wobei eine Zielfläche auf der Haut des Lebewesens mit aus einer Laser-Lichtquelle stammenden Teilchen beaufschlagt wird sowie einen zur Verwendung in dem Verfahren geeigneten Laser.

Es ist bekannt, mit Laser-Lichtquellen, die zu friedlichen Zwecken eingesetzt werden, Bearbeitungen verschiedener Materialien vorzunehmen. So können beispielsweise sehr feine und exakte Löcher in unterschiedlichste Materialien gebohrt werden. Ferner lassen sich Laser-Lichtquellen zum Punktschweißen einsetzen. Derartige Laser-Lichtquellen müssen eine vergleichsweise hohe Leistung haben, um die erforderliche Materialerwärmung durchführen zu können.

Ferner ist es auch bekannt, mit Lasern geringer Leistung das Wohlbefinden von Menschen oder Tieren zu steigern. Hierzu wird eine Mehrzahl von nebeneinander angeordneten Einzellasern mit geringer Leistung verwendet. Diese Laser-Lichtquellen-Anordnung wird in unmittelbare Nachbarschaft der Zielfläche gebracht. Dort erzeugt die Einwirkung der Laserstrahlung ein angenehmes Wärmegefühl. Insbesondere sollen Pferde -beispielsweise in der Regenerationsphase nach einer Verletzung - durch mehrfache tägliche Beaufschlagung mit derartiger Laserstrahlung für bis zu einer halben Stunde - schneller auf Hochleistung gebracht werden können. Nachteilig bei diesem bekannten Verfahren zur Leistungssteigerung ist es, daß die Laser-Lichtquelle in vergleichsweise nahen Kontakt mit der Zielfäche gebracht werden muß. Insbesondere vergleichsweise sensible Hochleistungspferde werden in ihrem Wohlbefinden durch derartige Maßnahmen gestört, so daß der gewünschte Effekt ins Gegenteil verkehrt wird.

Die Erfindung löst die Aufgabe, ein Verfahren zur Steigerung des Wohlbefindens eines Lebewesens gemäß der eingangs genannten Art zu - schaffen, das schneller und wirksamer arbeitet und zur Steigerung des Wohlbefindens bereits in kürzester Zeit beiträgt.

Zur Lösung dieser Aufgabe wird ein Verfahren vorgeschlagen, nachdem erfindungsgemäß die Laser-Lichtquelle entfernt von der Zielfläche angeordnet wird und dap eine insbesondere kurzgepulste, u.a. auch ultrakurzgepulste Laser-Lichtquelle mit einer hohen Austritts-Leistungsdichte derart verwendet wird, daß die Strahlung der Laser-Lichtquelle kurzzeitig eine hohe Leistung pro Flächeneinheit der Zielfläche auf die Zielfläche aufbringt. Vorteilhafte Weiterbildungen ergeben sich aus den Unteransprüche.

Dadurch, daß die Laser-Lichtquelle nicht in der Nähe des Lebewesens, also beispielsweise des Pferdes, dessen Leistungsbereitschaft über die Steigerung des Wohlbefindens gesteigert werden soll, sein muß, ist es möglich, die Laser-Lichtquelle so anzuordnen, daß sie nicht bemerkt wird. Zwar ist es bekannt, daß das Übertragen von Wärme ein wohliges Gefühl erzeugt. Jedoch reagieren insbesondere Pferde unwillig, wenn ein technisches Gerät mit ihnen in Kontakt gebracht wird.

Es hat sich gezeigt, daß die Leistungsbereitschaft eines Pferdes insbesondere durch die erfindungsgemäß aufgebrachte Strahlung gesteigert werden kann. Dadurch, daß eine vergleichsweise hohe Leistung pro Flächeneinheit kurzzeitig aufgebracht wird, werden verschiedene Vorteile erreicht. Zum einen wird die Eindringtiefe der Strahlung wesentlich vergrößert. Dadurch werden auch Gewebeschichten erreicht, die bislang nicht erreichbar waren. Des weiteren wird eine für die Steigerung des Wohlbefindens günstigere Verteilung der Strahlungsarten erreicht. Der Anteil der elektromagnetischen Strahlung sowie der Anteil der Phononen wird erhöht, wobei die Art der erzeugten Strahlung natürlich auch von der Art des verwendeten Lasers abhängt. Während die Photonenstatistik eines Lasers ohnehin für die gewünschte Wirkung günstiger als die thermischer Lichtquellen ist, zeigte es sich, daß die Strahlung bei Anwendung des erfindungsgemäßen Verfahrens Irritationen in den Zellen des Gewebes unterhalb der Zielfläche erzeugt. Diese Irritationen sind besonders günstig für die Erreichung des gewünschten Zwecks. Die mechanischen Irritationen sind auf Phononen zurückzuführen, die durch Energiesprünge erzeugt werden. Dies ist folgendermaßen zu erklären:

Ein eindringendes Photon weist eine bestimmte Eigenenergie auf - beispielsweise 1,04 eV. Durch diese Energie werden Elektronen eines Atoms bzw. eines Moleküls auf ein höheres Niveau gepumpt. Dieses energetisch ungünstigere Niveau bleibt nur sehr kurz bestehen; das Elektron gelangt in Einzelsprüngen auf sein Normalniveau zurück. Die Höhe eines jeden Einzelsprungs entspricht einer bestimmten Frequenz bzw. Energie und kann in eV ausgedrückt werden. Bestimmte Einzelsprünge liegen im Wellenbereich des Schalls, andere im Wellenbereich elektromagnetischer Strahlung. Die Phononen und die elektromagnetische Strahlung regen so insgesamt die Zelle an, was zu der gewünschten Wirkung führt.

Wichtig ist ferner die kurzzeitige Beaufschlagung mit hoher Leistung, die sich in vorteilhafter Weise bei Verwendung nur einer Lasereinheit erreichen läßt. Dadurch werden die gesamten Zellen unterhalb der Zielfläche gleichzeitig angeregt, was die entsprechende Wirkung verstärkt. Insgesamt kann so beispielsweise ein Pferd in der Regenerationsphase nach einer Verletzung in vergleichsweise kurzer Zeit auf Hochleistung gebracht werden.

Ein gewünschter Nebeneffekt besteht darin, daß bei chronischen Leiden der Pferde, beispielsweise Rheuma oder Gicht, die auftretenden Schmerzen offenbar weniger stark empfunden werden bzw. nahezu ausgeschaltet werden können und auch Gewebeverletzungen offenbar schneller heilen.

Ferner wird durch das gesteigerte Wohlbefinden offenbar insgesamt der Allgemeinzustand eines Lebewesens wie eines Pferdes verbessert, so daß ggf. etwaige Leiden oder Verletzungen - schneller abklingen.

Gewünschtenfalls kann der aus der Laser-Lichtquelle stammende Laserstrahl durch ein optisches System geleitet werden. Es ist aber auch möglich, die natürliche Divergenz des Laserstrahls auszunutzen, um eine Anpassung an die Größe der Zielfläche vorzunehmen.

So ist es möglich, die Laser-Lichtquelle mit einem Übertragungselement, wie z.B. einem Lichtleiter oder einem faseroptischen Lichtleiter, zu koppeln, wobei das Übertragungselement auch flexibel ausgebildet sein kann. Es ist ferner möglich, mehrere Laser-Lichtquellen in einem faseroptischen Lichtleiter zusammenzufassen, wenn kleine Austrittsflächen gewünscht werden, was besonders vorteilhaft bei kleinen Zielflächen der Fall ist, so daß z.B. eine sehr kleine Austrittsfläche erhalten wird, wenn eine Lichtleitfaser mit einem Durchmesser von 0,3 mm verwandt wird. Die Verwendung flexibler Übertragungselemente, insbesondere mit kleinen Austrittsflächen, hat den Vorteil, daß Zielflächen in tiefliegenden Körperhöhlen erreichbar sind und ohne das Lebewesen in besondere Positionen bringen zu müssen können mittels des flexiblen Übertragungselementes alle Körperstellen erreicht werden.

Als günstig hat sich beispielsweise eine Zielfläche mit einer Kantenlänge von 10 mm erwiesen, die mit einem Laser aus 2,5 m Entfernung bestrahlbar ist. Die Entfernung der Laser-Lichtquelle von der Zielfläche beträgt wenige Millimeter bis 5 m.

Ein weiterer Vorteil besteht darin, daß durch die kurzzeitige Einwirkung der Lasertstrahlung auf die Zielfläche keine Gewebeveränderungen erzeugt werden. Vielmehr wird eine Vielzahl von Zellen zugleich mechanisch irritiert, was dazu führt, daß in allen Fällen gleichmäßig eine Abwehrreaktion gegen die mechanische Irritation stattfindet. Diese wiederum ist günstig für das Wohlbefinden des Pferdes.

Weitere Vorteile, Einzelheiten und Merkmale ergeben sich aus der nachfolgenden Beschreibung eines Ausführungsbeispiels.

In einer Versuchsanordnung wurde ein Laser mit einer Leistung von 100 W verwendet. Diese wurde extrem kurzzeitig angesteuert, wobei der ansteuernde Stromimpuls eine Länge von nur 10 bis 12 ns aufwies. Der Laser hatte eine Austrittsfläche von 0,3 x 0,3 mm². Über ein optisches System mit einer Konvexlinse wurde die Zielfläche auf 10 mm x 10 mm bei einer Entfernung von 2,5 m zwischen der Laser-Lichtquelle und der Zielfläche eingestellt. Die Strahlung war innerhalb der Zielfläche völlig gleichmäßig verteilt. So behandelte Pferde, insbesondere solche, die zuvor an Schmerzen litten, fühlten sich offenbar wohl, denn ihre Leistung lag deutlich über den Erwartungen. Die Einwirkungszeit betrug etwa 1 bus 2 min, und zwar ein-bis zweimal täglich. Ferner war es möglich. durch die Steigerung des Wohlbefindens bzw. die Reduzierung von Schmerzen, die Regenerationszeit erheblich zu verkürzen. Die verwendete Wellenlänge der Photonen betrug 904 nm bzw. 1,04 eV. Die einminütige Einwirkung der Strahlung auf die Zielfläche führte dabei nicht zu einer nennenswerten Temperaturerhöhung an der betreffenden Stelle.

Die Erfindung läßt sich besonders gut an Pferderennbahnen einsetzen. Insbesondere können auch die Pferdebesitzer selbst - also nicht nur Tierärzte - die Erfindung nach entsprechender Einweisung einsetzen.

## Ansprüche

1. Verfahren zur Steigerung des Wohlbefindens eines Lebewesens, wobei eine Zielfläche auf der Haut des Lebewesens mit aus einer Laser-Lichtquelle stammenden Teilchen beaufschlagt wird, dadurch gekennzeichnet, daß die Laser-Lichtquelle entfernt von der Zielfläche angeordnet wird und daß eine insbesondere kurzgepulste , u.a. auch ultrakurzgepulste Laser-Lichtquelle mit einer hohen Austritts-Leistungsdichte derart verwendet wird, daß die Strahlung der Laser-Lichtquelle kurzzeitig eine hohe Leistung pro Flächeneinheit der Zielfläche auf die Zielfläche aufbringt.

2. Verfahren nach Anspruch 1, dadurch gekennzeichnet, daß die Austrittsfläche des Laserstrahles aus der Laser-Lichtquelle möglichst klein gehalten wird, insbesondere etwa 0,3 x 0,3 mm² beträgt.

3. Verfahren nach Anspruch 1 und 2, dadurch gekennzeichnet, daß für Zielflächen in einer Körperhöhle oder für Zielflächen, die um einen Winkel versetzt zur Laser-Lichtquellen-Strahlungsrichtung angeordnet sind, die Laser-Lichtquelle mit einem Übertragungselement, wie einem Lichtleiter oder einem faseroptischen Lichtleiter, verbunden wird, wobei das Übertragungselement auch flexibel ausgebildet sein kann.

4. Verfahren nach einem der vorhergehenden Ansprüche, dadurch gekennzeichnet, daß die Entfernung der Laser-Lichtquelle von der Zielfläche wenige Millimeter bis 5 m, vorzugsweise etwa 2 bis 5 m beträgt.

5. Verfahren nach einem der vorhergehenden Ansprüche, dadurch gekennzeichnet, daß die Divergenz des aus der Laser-Lichtquelle austretenden Laserstrahls verwendet wird, um eine Anpassung an die Größe der Zielfläche vorzunehmen.

6. Verfahren nach einem der vorhergehenden Ansprüche, dadurch gekennzeichnet, daß eine Laser-Lichtquelle mit einer Abgabeleistung von etwa 50 bis 350 W verwendet wird.

7. Verfahren nach einem der vorhergehenden Ansprüche, dadurch gekennzeichnet, daß die Laser-Lichtquelle ein-bis dreimal am Tag für 30 sec bis 4 Minuten angewandt wird.

8. Laser zur Verwendung in einem Verfahren gemäß einem der vorhergehenden Ansprüche, dadurch gekennzeichnet, daß die Laser-Lichtquelle nur eine Laser-Einheit aufweist oder daß eine Mehrfachanordnung von Laser-Lichtquellen vorgesehen ist, wobei die Laser-Lichtquellen mittels eines faseroptischen Lichtleiters zu einer kleinen Austrittsfläche vereinigt werden.

9. Laser nach Anspruch 8, dadurch gekennzeichnet, daß die Laserstrahlung eine Wellenlänge von etwa 904 nm aufweist.

10. Laser nach Anspruch 8 oder 9, dadurch gekennzeichnet, daß der für die Anregung des Lasers verwendete Stromimpuls weniger als 15 ns, insbesondere 10 ns, dauert.

11. Laser nach einem der vorhergehenden Ansprüche 8 bis 10 , dadurch gekennzeichnet, daß die abgegebene Lichtimpulsbreite im Bereich von einigen ns liegt.

| | EUROPÄISCHER TEILRECHERCHENBERICHT, der nach Regel 45 des Europäischen Patent- übereinkommens für das weitere Verfahren als europäischer Recherchenbericht gilt | Nummer der Anmeldung |
| Europäisches Patentamt | | EP 86 11 3792 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-2 577 425 (BOUCHLAGEM)<br><br>* Seite 5, Zeilen 19-29; Seite 6, Zeilen 8-24 *<br><br>--- | 1,3,4,<br>8,9 | A 61 N 5/06 |
| E | WO-A-86 06 642 (CLARKE)<br><br>* Seite 22, Zeilen 1-12 *<br><br>--- | 1,3,4,<br>8-11 | |
| A | EP-A-0 143 185 (CARL ZEISS)<br><br>* Seite 8, Zeilen 3-15 *<br><br>----------- | 1,4,5,<br>8,10,<br>11 | |

**RECHERCHIERTE SACHGEBIETE (Int. Cl.4)**

A 61 N

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: **8-11**
Unvollständig recherchierte Patentansprüche: **1-7**
Nicht recherchierte Patentansprüche:
Grund für die Beschränkung der Recherche:

Verfahren zur chirurgischen oder therapeutischen Behandlung des menschlichen oder tierischen Körpers (Siehe Art. 52(4) des Europäischen Patentübereinkommens).

| Recherchenort | Abschlußdatum der Recherche | Prüfer |
|---|---|---|
| Den Haag | 09-06-1987 | LEMERCIER |